# EUROPEAN PATENT APPLICATION

(11) **EP 3 730 064 A1**
(43) Date of publication of application: **28.10.2020**
(21) Application number: 20171227.0
(22) Date of filing: 24.04.2020
(51) Int. Cl.: A61B 17/00, A61B 17/04, A61B 17/06, A61B 17/29, A61B 90/00, A61B 90/50

(54) **PORT SITE INCISION CLOSURE DEVICE**

(30) Priority: 26.04.2019 US 201962838956 P; 31.03.2020 US 202016835633
(71) Applicant: Covidien LP, Mansfield, MA 02048 (US)
(72) Inventor: BHALLA, Inderjeet Singh, 500075 Telangana (IN); PATNAIK, Santosh Kumar, AP 500084 Hyderabad (IN); KUMAR, Neeraj, UP 201301 NOIDA (IN)
(74) Representative: Maschio, Antonio

(57) **Abstract**

A method and device for closing a port site incision after a minimally invasive surgical procedure is performed includes an elongate body and a tool assembly supported on a distal portion of the elongate body. The tool assembly includes a suture needle that is moved between an active position in which a tip of the suture needle is exposed and an active position in which the tip of the needle is received within a shield supported on the elongate body by an actuation member. The actuation member is connected to the suture needle by a linkage that maintains a longitudinal axis of the suture needle parallel to the longitudinal axis of the elongate body as the tool assembly is moved between the active and inactive positions.

## Description

### BACKGROUND

### 1. Technical Description

The present disclosure is directed to a closure device for closing incisions, and more particularly, to a closure device for closing port site incisions after minimally invasive surgical procedures.

### 2. Background of Related Art

Minimally invasive surgical procedures including laparoscopic, endoscopic, and arthroscopic surgical procedures are performed through cannulas positioned through small port site incisions in a body wall of a patient that access a surgical site. Minimally invasive surgical procedures impose less risk of infection and result in shortened hospital stays as compared to traditional open surgical procedures.

After a minimally invasive surgical procedure is completed, the port site incisions provided to access the surgical site must be closed or sutured. Current procedures for closing port site incisions after minimally invasive surgical procedures are highly skill dependent and typically involve directing a curved needle through a small port and through fascial and muscular layers of tissue while attempting to avoid contact with internal body organs. Procedures for closing port site incisions in the abdominal wall of obese patients can be especially difficult after a laparoscopic surgical procedure.

A continuing need exists in the surgical arts for an improved closure device for sealing or closing a port site incision that is simple in construction and easy to use.

### SUMMARY

One aspect of the present disclosure is directed to an incision closure device including an elongate body, and actuation member, and a tool assembly. The elongate body has a proximal portion and a distal portion and defines a channel that extends from the proximal portion to the distal portion. The elongate body defines a longitudinal axis and supports a shield. The tool assembly is supported on the distal portion of the elongate body and includes a suture needle, a housing and first and second links. The suture needle has a proximal portion having a tip configured to penetrate tissue and a distal portion. The distal portion of the suture needle is pivotably secured to the first and second links of the tool assembly and the first and second links are pivotably secured to the housing. The actuation member is positioned within the channel of the elongate body and is coupled to the first and second links such that the actuation member is movable from a retracted position to an advanced position to move the suture needle from an inactive position in which the tissue penetrating tip of the suture needle is positioned within the shield to an active position in which the tissue penetrating tip is positioned outwardly of the shield.

In embodiments, the suture needle defines a longitudinal axis that is substantially parallel to the longitudinal axis of the elongate body in the inactive position and in the active position.

In some embodiments, each of the first and second links defines an elongate slot and the actuation member is coupled to the first and second links by pivot pins that are positioned within the elongate slots.

In certain embodiments, the distal portion of the actuation member includes a bracket having spaced side walls defining a channel, wherein the first and second links are received within the channel.

In embodiments, the proximal portion of the suture needle includes a yoke defining a yoke channel, wherein the first and second links are secured within the yoke channel.

In some embodiments, the closure device includes a handle assembly, wherein the elongate body extends distally from the handle assembly.

In certain embodiments, the handle assembly includes a drive member and a trigger, wherein the drive member is coupled to a proximal end of the actuation member and the trigger is coupled to the drive member such that actuation of the trigger causes movement of the actuation member between the retracted and advanced positions.

In embodiments, the handle assembly includes a biasing member that is positioned to engage the drive member to urge the actuation member towards the retracted position.

In some embodiments, the handle assembly includes a housing and the housing defines a recess that receives the drive member.

In certain embodiments, the housing includes a detent positioned within the recess and the drive member includes first and second concavities, wherein the first concavity is positioned to receive the detent when the actuation member is in the retracted position and the second concavity is positioned to receive the detent when the actuation member is in the advanced position.

In embodiments, the trigger includes first and second triggers that are coupled to the drive member and movable in a scissor-like motion to move the actuation member from the retracted position to the advanced position.

In some embodiments, the triggers are coupled to the drive member by first and second drive links.

Another aspect of the present disclosure is directed to a method of closing an incision including inserting a tool assembly and an elongate body of a closure device through an incision with the tool assembly in an inactive position, in which a tissue penetrating tip of a suture needle is positioned within a shield on the elongate body, to position the tool assembly within a body cavity; advancing an actuation member of the closure device to move the tool assembly from the inactive position to an active position in which the tissue penetrating tip of the suture needle is removed from the shield and positioned beneath a first side of the incision; retracting the closure device within the incision to move the tissue penetrating tip of the suture needle and a first end of a suture supported on the suture needle through tissue; grasping the first end of the suture and pulling the first end of the suture from the incision; inserting the tool assembly back into the body cavity; rotating the tool assembly to position the tissue penetrating tip of the suture needle beneath a second side of the incision; retracting the closure device within the incision to advance the tissue penetrating tip of the suture needle and a second end of the suture supported on the suture needle through tissue on the second side of the incision; grasping the second end of the suture and pulling the second end of the suture from the incision; and applying tension to the first and second ends of the suture to close the incision.

In embodiments, the method includes moving the tool assembly from the active position to the inactive position after inserting the tool assembly back into the body cavity.

In embodiments, moving the tool assembly from the inactive position to the active position after rotating the closure device to position the suture needle beneath the second side of the incision.

### BRIEF DESCRIPTION OF THE DRAWINGS

Various embodiments of the presently disclosed port site incision closure device are described herein below with reference to the drawings, wherein:
FIG. 1 is a side perspective view of an exemplary embodiment of the presently disclosed port site incision closure device with a suture needle of a tool assembly of the closure device in an inactive position;
FIG. 2 is a side perspective view of a handle assembly of the closure device shown in FIG. 1 in a non-actuated position with a handle housing section removed;
FIG. 3 is a side perspective, exploded view of the closure device shown in FIG. 1;
FIG. 3A is a side perspective view of a drive member of a handle assembly of the closure device shown in FIG. 3;
FIG. 4 is an enlarged view of the indicated area of detail shown in FIG. 3;
FIG. 5 is a cross-sectional view taken along section line 5-5 of FIG. 1;
FIG. 6 is an enlarged view of the indicated area of detail shown in FIG. 5;
FIG. 7 is an enlarged view of the indicated area of detail shown in FIG. 1;
FIG. 8 is an enlarged view of the indicated area of detail shown in FIG. 5;
FIG. 9 is a side perspective view of the closure device of FIG. 1 with the handle assembly in an actuated position and the suture needle of the tool assembly in the active position;
FIG. 10 is a cross-sectional view taken along section line 10-10 of FIG. 9;
FIG. 11 is an enlarged view of the indicated area of detail shown in FIG. 10;
FIG. 12 is a side view of a distal portion of the closure device shown in FIG. 1 with the tool assembly positioned through a port site incision in a body cavity with the suture needle of the tool assembly in the inactive position and a suture attached to the suture needle and extending through the port site incision;
FIG. 13 is a side view of the distal portion of the closure device shown in FIG. 12 with the tool assembly positioned within the body cavity and the suture needle of the tool assembly in the active position with the suture needle positioned beneath the tissue defining a first side of the port site incision and the suture extending through the port site incision;
FIG. 14 is a side view of the distal portion of the closure device shown in FIG. 13 with the tool assembly partially retracted into the port site incision, the suture needle in the active position and extending through the tissue defining the first side of the port site incision and one end of the suture extending through the tissue defining the first side of the port site incision;
FIG. 15 is a side view of the distal portion of the closure device shown in FIG. 14 with the tool assembly repositioned within the body cavity, the suture needle in the active position, one end of the suture extending through the tissue defining the first side of the port site incision, and the other end of the suture extending through the port site incision;
FIG. 16 is a side view of the distal portion of the closure device shown in FIG. 15 with the tool assembly rotated to a position in which the suture needle of tool assembly is positioned beneath a second side of the tissue defining the port site incision;
FIG. 17 is a side view of the distal portion of the closure device shown in FIG. 16 with the tool assembly partially retracted into the port site incision, the suture needle in the active position and extending through the tissue defining the second side of the port site incision, and one end of the suture extending through the tissue defining the first side of the port site incision and the other end of the suture extending through the tissue defining the second side of the port site incision;
FIG. 18 is a side cross-sectional view of the tissue defining the port site incision with the one end of the suture extending through the tissue defining the first side of the port site incision and the other end of the suture extending through the tissue defining the second side of the port site incision; and
FIG. 19 is a side cross-sectional view of the tissue defining the port site incision shown in FIG. 18 with the suture tensioned to close the port site incision.

### DETAILED DESCRIPTION OF EMBODIMENTS

The presently disclosed closure device will now be described in detail with reference to the drawings in which like reference numerals designate identical or corresponding elements in each of the several views. However, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "endoscopic" is used generally used to refer to endoscopic, laparoscopic, arthroscopic, and/or any other procedure conducted through small diameter incision or cannula. Further, the term "clinician" is used generally to refer to medical personnel including doctors, nurses, and support personnel.

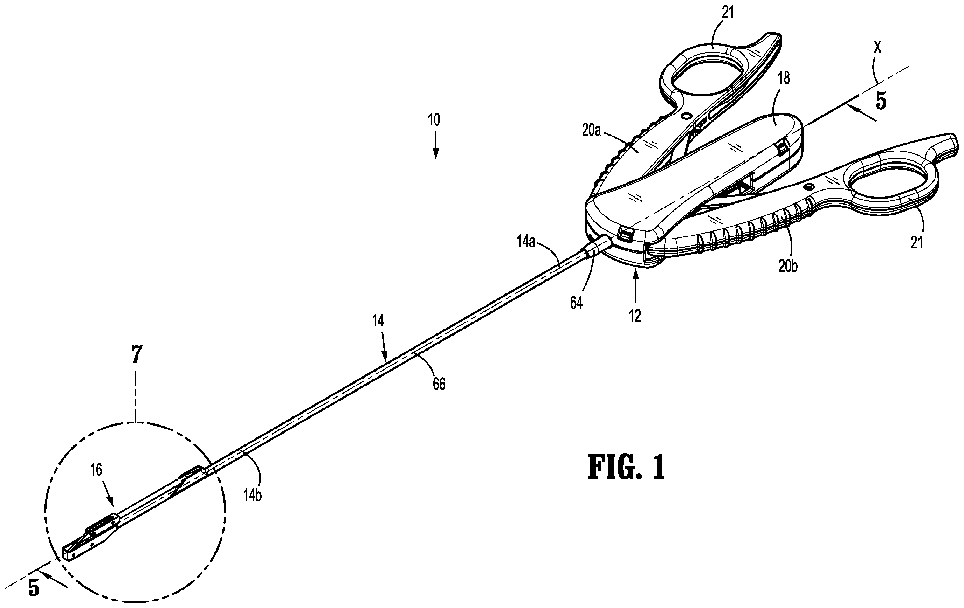

FIG. 1 illustrates an exemplary embodiment of the presently disclosed port site incision closure device shown generally as closure device 10. The closure device 10 includes a handle assembly 12, an elongate body 14 defining a longitudinal axis "X", and a tool assembly 16. The handle assembly 12 includes a central housing 18 and a pair of triggers 20 that are supported on the housing 18 for movement in a scissor-like fashion to actuate the tool assembly 16. Alternately, other types of actuators or triggers can be provided to actuate the tool assembly 16. In embodiments, each of the triggers 20a, 20b includes a finger loop 21 for receiving a finger of a clinician's hand (not shown). The elongate body 14 has a proximal portion 14a that extends distally from the central housing 18 of the handle assembly 12 and a distal portion 14b that supports the tool assembly 16.

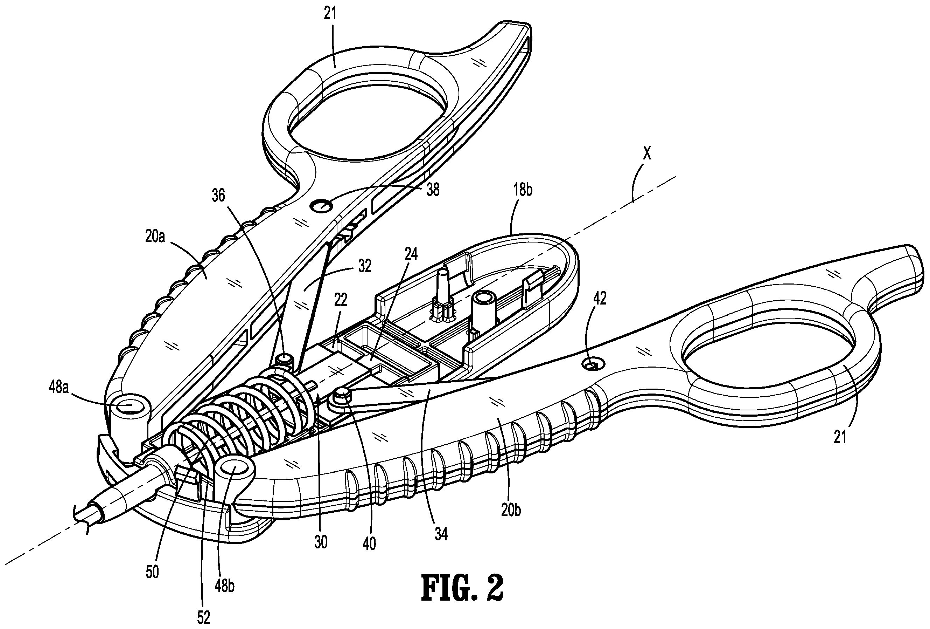

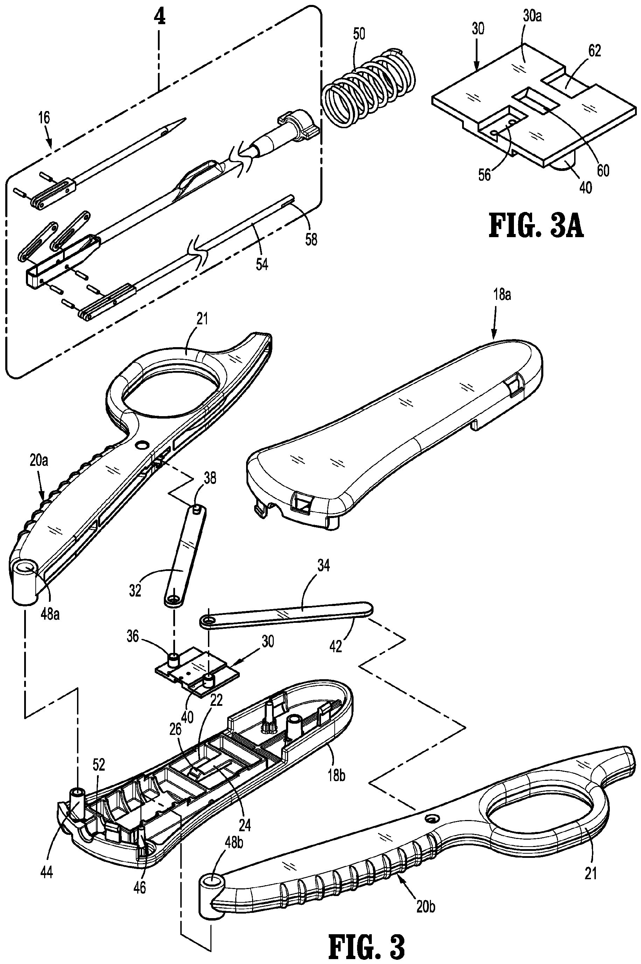

Referring to FIGS. 2 and 3, the housing 18 may be formed as a unitary component or, as depicted in FIG. 3, as two separate half components 18a, 18b, that are coupled to one another by one or more suitable coupling methods, e.g., screws, adhesives, welding or the like. The housing 18 defines a recess 22 that includes a rib 24 that extends longitudinally within the housing 18. The rib 24 includes a detent 26 (FIG. 3) that is positioned on a distal portion of the rib 24 and is described in further detail below.

The housing 18 supports a drive member 30 that is coupled to the triggers 20a, 20b by first and second drive links 32, 34, respectively. The drive link 32 has a distal end that is pivotally coupled to the drive member 30 by a pivot member 36 and a proximal end that is pivotally coupled to a central portion of the trigger 20a by a pivot member 38. Similarly, the drive link 34 has a distal end that is pivotally coupled to the drive member 30 by a pivot member 40 and a proximal end that is pivotally coupled to a central portion of the trigger 20b by a pivot member 42.

The distal portions of the triggers 20a, 20b are pivotally secured to a distal portion of the housing 18 by pivot members 44, 46 (FIG. 3), respectively. In embodiments, the triggers 20a, 20b each define an opening 48a, 48b for receiving a respective one of the pivot members 44, 46. The pivot members 44, 46 axially fix the distal portion of the triggers 20a, 20b to the housing 18 while permitting pivotal movement of the triggers 20a, 20b in relation to the housing 18.

The drive links 32, 34 are positioned to move the drive member 30 distally within the housing 18 when the triggers 20a, 20b are compressed together from a retracted position to an advanced position. More specifically, when the triggers 20a, 20b are compressed together, the links 32, 34 are moved from a position at an angle to the longitudinal axis "X" of the elongate body 14 towards a position aligned with the longitudinal axis "X". As the links 32, 34 move towards a position aligned with the longitudinal axis "X", the drive member 30 is advanced within the housing 18 from a retracted position to an advanced position.

The handle assembly 12 includes a biasing member 50 that is positioned within the housing 18. The biasing member 50 has a distal end that is supported against an inner distal wall 52 of the housing 18 and a proximal end that is engaged with the distal end of the drive member 30. The biasing member 50 urges the drive member 30 towards the retracted position to urge the triggers 20a, 20b via the links 32, 34 apart (FIG. 2).

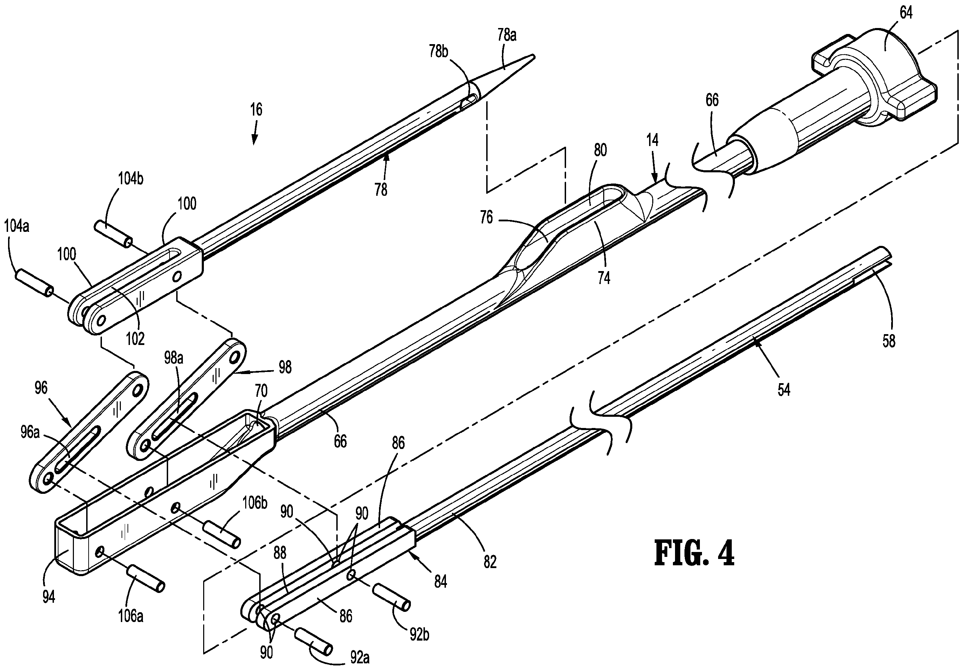

Referring to FIG. 3A and 4, the underside 30a of the drive member 30 (FIG. 3A) is configured to engage a proximal end of an actuation member 54. In embodiments, the distal portion of the drive member 30 defines a cutout 56 that is received within a slot 58 defined in a proximal portion of the actuation member 54. In embodiments, the proximal portion of the actuation member 30 is secured to the drive member 30 using any known fastening technique including, welding, pins, rivets, or the like, such that longitudinal movement of the drive member 30 is translated into longitudinal movement of the actuation member 54.

The underside 30 of the drive member 30 also defines a first concavity 60 and a second concavity 62. The first and second concavities 60, 62 are longitudinally aligned with each other and with the detent 26 (FIG. 3) of the half component 18b (FIG. 3) of the housing 18. The detent 26 is received within the first concavity 62 of the drive member 30 when the drive member 30 is in the retracted position and received in the second concavity 62 of the drive member 30 when the drive member 30 is in the advanced position as described below. Receipt of the detent 26 within the first and second concavities 60, 62 retains the drive member 30 and the actuation member 54 in the retracted and advanced positions, respectively.

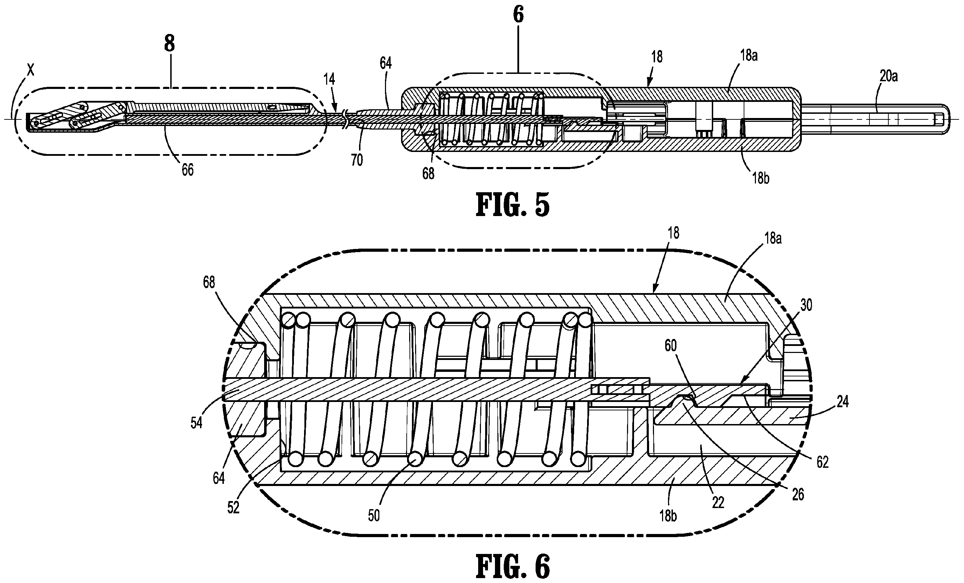

Referring to FIG. 4-6, the elongate body 14 includes a proximal portion supporting a coupling member 64 and a distal portion 66. The coupling member 64 is fixedly supported within a recess 68 (FIG. 5) in a distal portion of the housing 18 to axially fix the elongate body 14 in relation to the housing 18. The distal portion 66 of the elongate body 14 supports the tool assembly 16. The coupling member 64 and distal portion 66 of the elongate body 14 are hollow and define a channel 70 (FIG. 5) that receives the actuation member 54. The channel 70 is dimensioned to facilitate axial movement of the actuation member 54 between retracted and advanced positions in response to axial movement of the drive member 30.

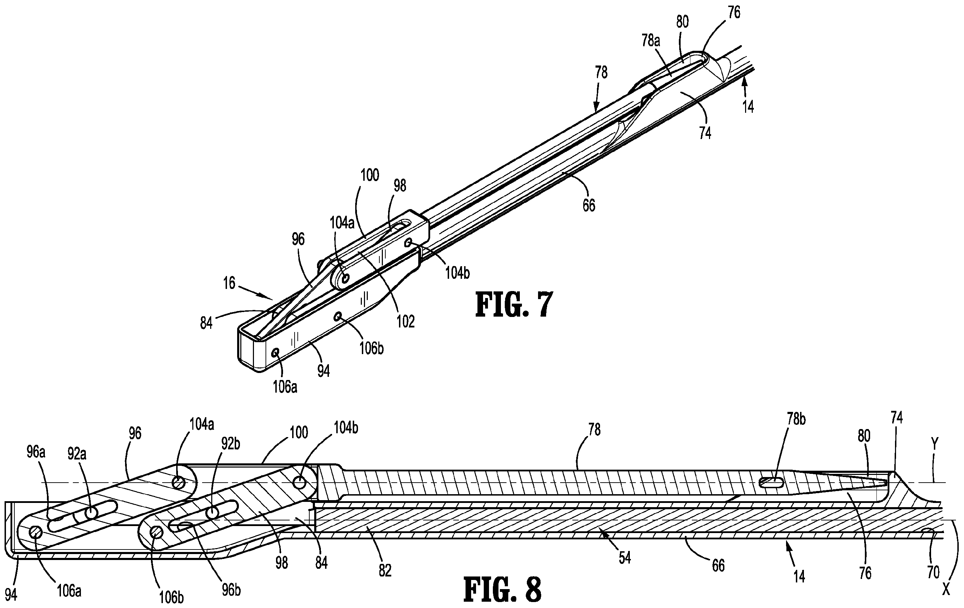

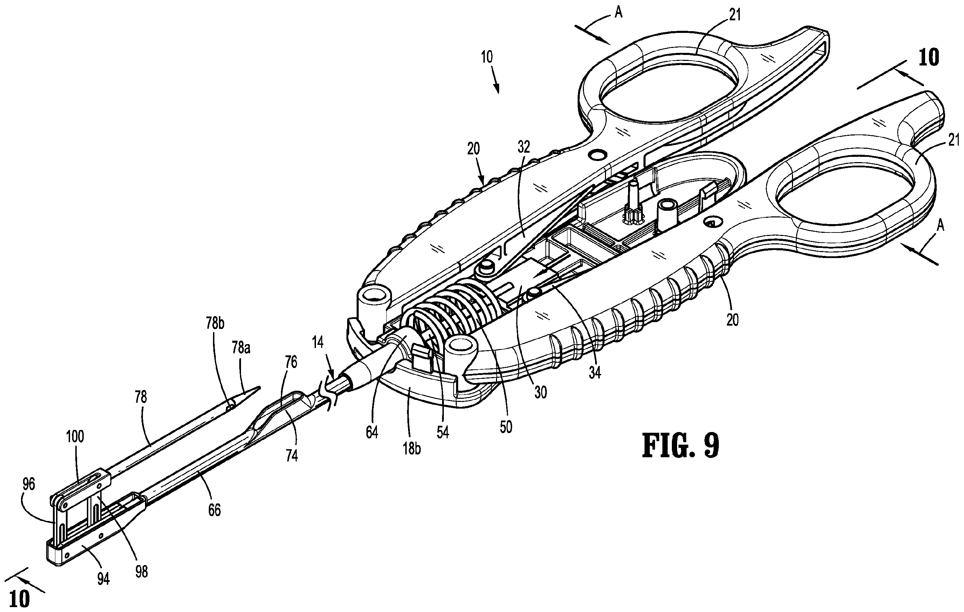

The distal portion 66 of the elongate body 14 defines a shield 74 (FIG. 4) that includes a side wall 80 that defines a cavity 76 that receives a tip 78a of a suture needle 78 of the tool assembly 16 when the suture needle 78 is in an inactive position (FIG. 8). The tip 78a is tapered to penetrate tissue and includes a suture opening 78b positioned adjacent the tapered tip 78a. The cavity 76 defined by the shield 74 extends in a direction parallel to the longitudinal axis "X" of the elongate body 14 and is positioned to receive the tip 78a of the suture needle 78 when the suture needle 78 moves from an active position (FIG. 9) to an inactive position (FIG. 8). When the tip 78a of the suture needle 78 is positioned within the cavity 76, the tip 78a of the suture needle 78 is guarded by the side wall 80 of the shield 74 to protect a clinician from needle stick injuries during handling of the closure device 10 before and after use.

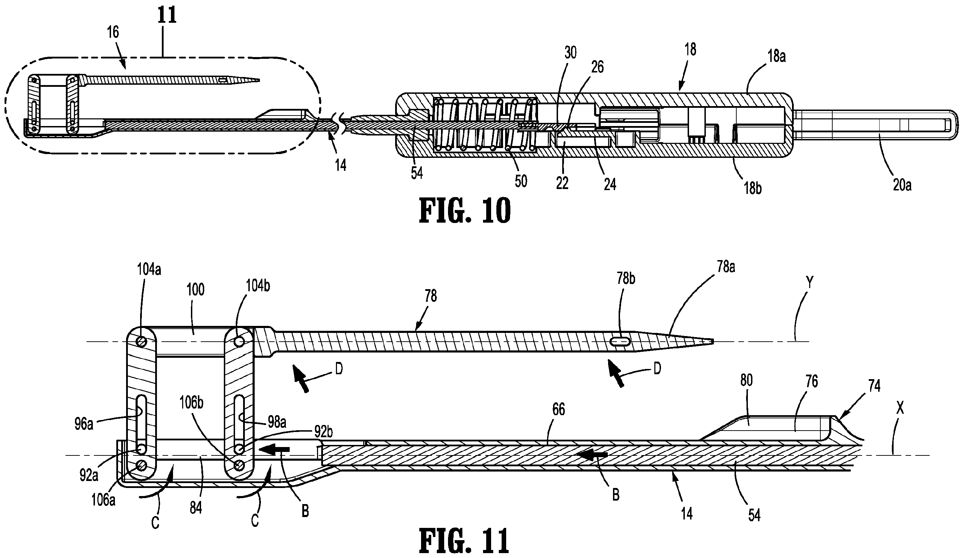

Referring to FIGS. 4, 7 and 8, the actuation member 54 is movably positioned within the channel 70 (FIG. 8) of the elongate body 14 between a retracted position (FIG. 8) and an advanced position (FIG. 11). The actuation member 54 includes a distal portion 82 having a bracket 84 (FIG. 4) with spaced side walls 86 that define a channel 88. The side walls 86 each define openings 90 that receive pivot pins 92a, 92b as described in further detail below.

The tool assembly 16 includes a housing 94 supported on the distal portion 14b of the elongate body 14, first and second links 96, 98, and the suture needle 78. Each of the first and second links 96, 98 defines an elongate slot 96a, 98a. The proximal portion of the suture needle 78 forms a yoke 100 that defines a channel 102 that receives distal ends of the first and second links 96, 98. The yoke 100 of the suture needle 78 is coupled to the bracket 84 of the actuation member 54 by the links 96, 98. More specifically, each of the links 96, 98 has a proximal end that is pivotally coupled to the yoke 100 of the suture needle 78 by pivot members 104a, 104b and a distal end that is pivotally coupled to the housing 94 of the tool assembly 16 by pivot pins 106a, 106b. In addition, the first and second links 96, 98 are coupled to the bracket 84 of the actuation member 54 by the pivot pins 92a, 92b which extend through the elongate slots 96a, 98a of the first and second links 96, 98, respectively.

Referring to FIGS. 5-8, when the handle assembly 12 is in the non-actuated position (FIG. 1), the drive member 30 is in its retracted position such that the actuation member 54 is in its retracted position. In the retracted position, the detent 26 (FIG. 6) formed on the rib 24 within the recess 22 of the housing 18 of the handle assembly 12 is received within the first concavity 60 (FIG. 6) of the drive member 30 to retain the drive member 30 and the actuation member 54 in their retracted positions. In the retracted position of the actuation member 54, the distal ends of the first and second links 96, 98 of the tool assembly 16 are pivoted downwardly towards the longitudinal axis "X" of the elongate body 14 to a position adjacent an outer surface of the distal portion 66 of the elongate body 14 to position the tool assembly 16 in the inactive position with the tip 78a of the suture needle 78 positioned within the shield 74 (FIG. 7). In the inactive position of the tool assembly 16, the suture needle 78 defines an axis "Y" (FIG. 8) that is substantially parallel to the longitudinal axis "X" of the elongate body 14.

Referring to FIGS. 9-11, when the triggers 20 are compressed towards each other in the directions indicated by arrows "A" in FIG. 9, the first and second drive links 32, 34 are driven distally to advance the drive member 30 within the housing 18 of the handle assembly 12 against the urging of the biasing member 50. The drive member 30 is coupled to the proximal end of the actuation member 54 such that advancement of the drive member 30 within the housing 18 advances the actuation member 54 within the elongate body 14 of the closure device 10. As the actuation member is advanced within the elongate body 14 in the direction indicated by arrows "B" in FIG. 11, the pins 92a, 92b are driven through the elongate slots 96a, 98a formed in the first and second links 96, 98, respectively to rotate the links 96, 98 in the direction indicated by arrows "C" in FIG. 11 about the pivot members 106a, 106b, respectively. As the first and second links 96, 98 rotate in the direction of arrows "C", the proximal ends of the first and second links 96, 98 move outwardly of the longitudinal axis "X" of the elongate body 14 to lift the suture needle 78 in the direction indicated by arrows "D" in FIG. 11 from its inactive position shown in FIG. 8 to its active position shown in FIG. 11. As illustrated, the pivot pins 104a, 104b that secure the distal end of the first and second links 96, 98 to the proximal portion of the suture needle 78 are spaced from each other and axially fixed in relation to the suture needle 78. As such, the longitudinal axis "Y" of the suture needle 78 remains substantially parallel to the longitudinal axis "X" of the elongate body 14 of the closure device 10 as the suture needle 78 is moved between its inactive and active positions.

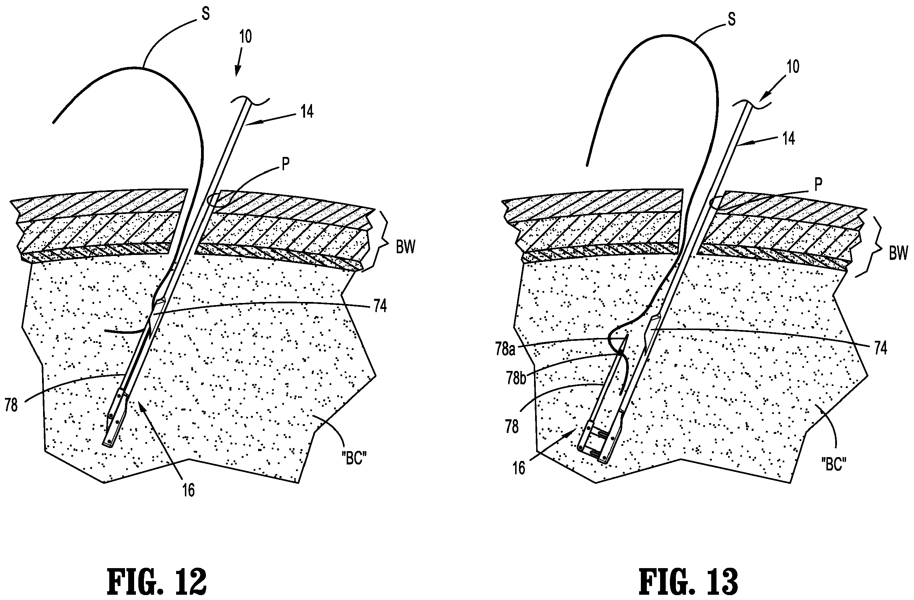

FIGS. 12-19 illustrate use of the presently disclosed closure device 10 for closing a port site incision "P" in a body wall "BW" of a patient after a minimally invasive surgical procedure. Referring to FIGS. 12 and 13, after a minimally invasive surgical procedure is completed, or any time it becomes necessary to close an incision "P" in a body wall "BW" of a patient, the elongate body 14 of the closure device 10 is inserted through the port site incision "P" in the body wall "BW" with a suture "S" secured to the suture needle 78 via the opening 78b to position the tool assembly 16 within a body cavity "BC". During insertion, the tool assembly 16 is retained in the inactive position (FIG. 12) with the tip 78a of the suture needle 78 positioned within the cavity 76 (FIG. 4) of the shield 74. After the tool assembly 16 is positioned within the body cavity "BC", the tool assembly 16 is moved from the inactive position (FIG. 12) to the active position (FIG. 13) by compressing or moving the triggers 20 (FIG. 1) towards each other to advance the actuation member 54 as discussed above. As the suture needle 78 moves towards the active position, the tip 78a of the suture needle 78 moves outwardly of the longitudinal axis "X" of the elongate body portion 14 and is removed from the cavity 76 of the shield 74. As shown, in the active position, the tip 78a of the suture needle 78 is directed towards the incision "P" and the handle assembly 12. At this point in the method, the suture "S" is positioned to extend through the incision "P".

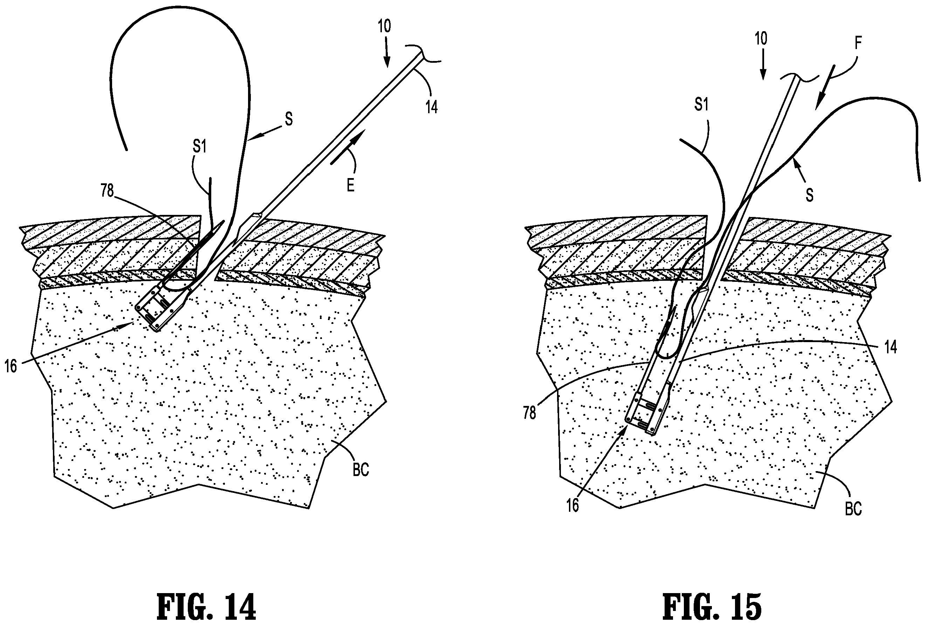

Referring to FIG. 14, after the suture needle 78 is in the active position, the closure device 10 can be tilted and partially withdrawn from within the incision "P" in the direction indicated by arrows "E" to move the tip 78a of the suture needle 78 and a first end "S1" of the suture "S" at an angle through fascial and muscular layers of tissue of the body wall "BW" adjacent one side of the incision "P". When the first end "S1" of the suture 36 is accessible to a clinician, the clinician grasps and pulls the first end "S1" through the incision "P".

Referring to FIG. 15, after the clinician has a firm grasp on the first end "S1" of the suture 36, the closure device 10 is inserted back through the incision "P" in the body wall "BW" in the direction indicated by arrow "F" to reposition the tool assembly 16 within the body cavity "BC". Once the tool assembly 16 is positioned fully within the body cavity "BC", the clinician may choose to return the tool assembly 16 to the inactive position to guard against inadvertently damaging internal organs during manipulation of the closure device 10.

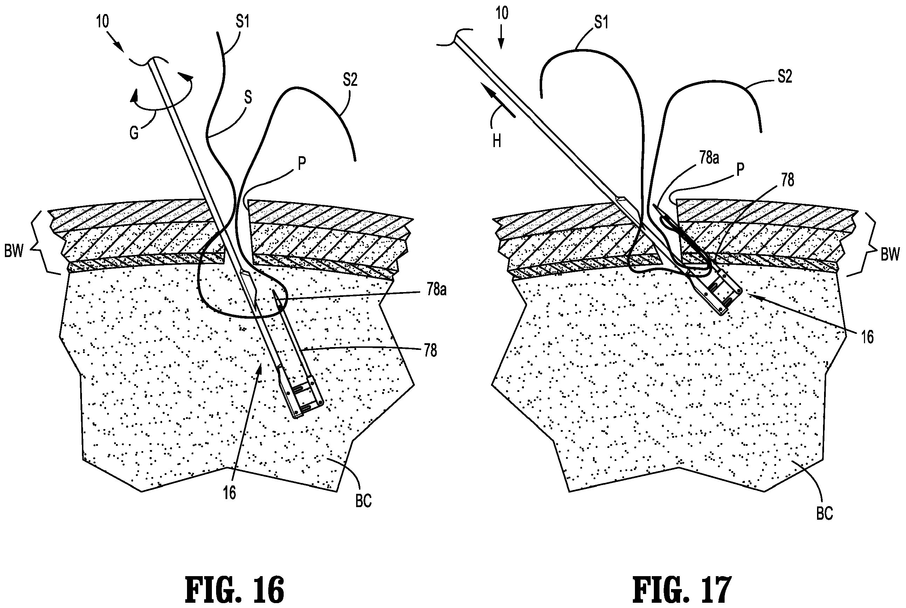

Referring to FIG. 16, after the tool assembly 16 of the closure device 10 is repositioned within the body cavity "BC", the closure device 12 can be rotated in the directions indicated by arrows "G" within the incision "P" to reposition the tip 78a of the suture needle 78 beneath an opposite side of the incision "P". If the closure device 10 is in the inactive position, the tool assembly 16 should be moved to the active position (FIG. 16) after the closure device 10 is rotated to the position shown in FIG. 16.

Referring to FIG. 17, after the closure device 10 is rotated to the position shown in FIG. 16, the closure device 10 can be tilted in an opposite direction and partially withdrawn from within the incision "P" in the direction indicated by arrows "H" to move the tip 78a of the suture needle 78 and a second end "S2" of the suture "S" at an angle through fascial and muscular layers of the body wall "BW" adjacent an opposite side of the incision "P" to position the second end "S2" of the suture "S" adjacent the opposite side of the incision "P". When the second end "S2" of the suture "S" is accessible to a clinician, the clinician grasps and pulls the second end "S2" of the suture "S" through the incision "P".

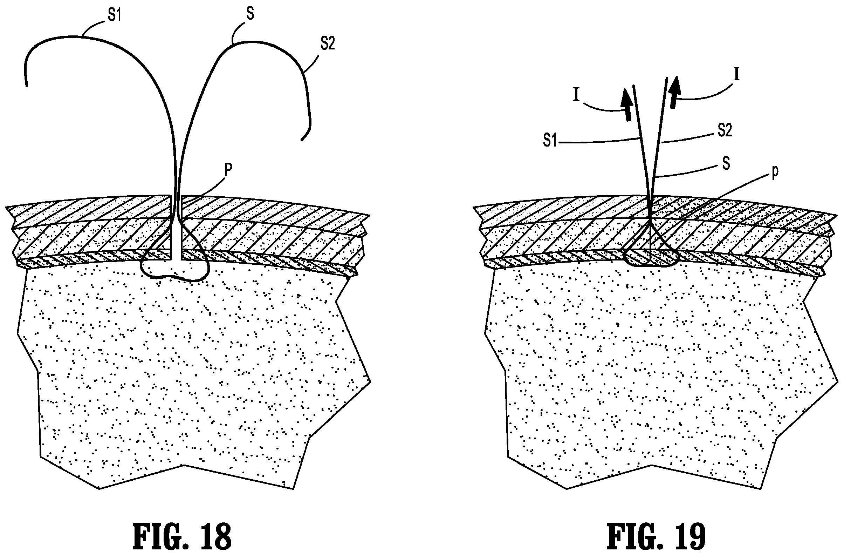

Referring to FIGS.18 and 19, after the clinician has a grasp on both ends "S1" and "S2" of the suture "S", the closure device 10 can be withdrawn from within the incision "P" and properly disposed of in a customary manner. Thereafter, the clinician can apply tension to the ends "S1" and "S2" of the suture "S" in the direction indicated by arrows "I" to close the incision "P".

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

The invention may be described by reference to the following numbered paragraphs:
1. An incision closure device comprising: an elongate body defining a longitudinal axis and having a proximal portion and a distal portion, the elongate body defining a channel that extends from the proximal portion to the distal portion and supporting a shield; a tool assembly supported on the distal portion of the elongate body, the tool assembly including a suture needle, a housing and first and second links, the suture needle having a proximal portion having a tip configured to penetrate tissue and a distal portion, the distal portion being pivotably secured to the first and second links of the tool assembly, the first and second links being pivotably secured to the housing; an actuation member positioned within the channel of the elongate body and coupled to the first and second links such that the actuation member is movable from a retracted position to an advanced position to move the suture needle from an inactive position in which the tissue penetrating tip of the suture needle is positioned within the shield to an active position in which the tissue penetrating tip is positioned outwardly of the shield.
2. The incision closure device of paragraph 1, wherein the suture needle defines a longitudinal axis that is substantially parallel to the longitudinal axis of the elongate body in the inactive position and in the active position.
3. The incision closure device of paragraph 1, wherein each of the first and second links defines an elongate slot and the actuation member is coupled to the first and second links by pivot pins that are positioned within the elongate slots.
4. The incision closure device of paragraph 3, wherein the distal portion of the actuation member includes a bracket having spaced side walls defining a channel, the first and second links being received within the channel.
5. The incision closure device of paragraph 4, wherein the proximal portion of the suture needle includes a yoke defining a yoke channel, the first and second links being secured within the yoke channel.
6. The incision closure device of paragraph 1, further including a handle assembly, wherein the elongate body extends distally from the handle assembly.
7. The incision closure device of paragraph 6, wherein the handle assembly includes a drive member and a trigger, the drive member being coupled to a proximal end of the actuation member and the trigger being coupled to the drive member such that actuation of the trigger causes movement of the actuation member between the retracted and advanced positions.
8. The incision closure device of paragraph 7, further including a biasing member positioned to engage the drive member to urge the actuation member towards the retracted position.
9. The incision closure device of paragraph 8, wherein the handle assembly includes a housing and the housing defines a recess that receives the drive member.
10. The incision closure device of paragraph 9, wherein the housing includes a detent positioned within the recess and the drive member includes first and second concavities, the first concavity being positioned to receive the detent when the actuation member is in the retracted position and the second concavity being positioned to receive the detent when the actuation member is in the advanced position.
11. The incision closure device of paragraph 10, wherein the trigger includes first and second triggers that are coupled to the drive member and movable in a scissor-like motion to move the actuation member from the retracted position to the advanced position.
12. The incision closure device of paragraph 11, wherein the triggers are coupled to the drive member by first and second drive links.
13. A tool assembly comprising: a suture needle, a housing, and first and second links, the suture needle having a proximal portion having a tip configured to penetrate tissue and a distal portion, the distal portion pivotably secured to the first and second links, the first and second links being pivotably secured to the housing, the first and second links movable from a first position to a second position to move the suture needle from an inactive position in which the tissue penetrating tip of the suture needle is positioned within the shield to an active position in which the tissue penetrating tip is positioned outwardly of the shield.
14. The tool assembly of paragraph 13, wherein the proximal portion of the suture needle includes a yoke defining a yoke channel, the first and second links secured within the yoke channel.
15. The tool assembly of paragraph 14, wherein the suture needle defines a first axis in the active position and a second axis in the inactive position, the first axis being parallel to the second axis.
16. The tool assembly of paragraph 13, wherein the first and second links each define an elongated slot, the elongated slots receiving a pivot member that is secured to the housing.
17. The tool assembly of paragraph 13, wherein the suture needle includes a suture opening.
18. A method of closing an incision comprising: inserting a tool assembly and an elongate body of a closure device through an incision with the tool assembly in an inactive position, in which a tissue penetrating tip of a suture needle is positioned within a shield on the elongate body, to position the tool assembly within a body cavity; advancing an actuation member of the closure device to move the tool assembly from the inactive position to an active position in which the tissue penetrating tip of the suture needle is removed from the shield and positioned beneath a first side of the incision; retracting the closure device within the incision to move the tissue penetrating tip of the suture needle and a first end of a suture supported on the suture needle through tissue; grasping the first end of the suture and pulling the first end of the suture from the incision; inserting the tool assembly back into the body cavity; rotating the tool assembly to position the tissue penetrating tip of the suture needle beneath a second side of the incision; retracting the closure device within the incision to advance the tissue penetrating tip of the suture needle and a second end of the suture supported on the suture needle through tissue on the second side of the incision; grasping the second end of the suture and pulling the second end of the suture from the incision; and applying tension to the first and second ends of the suture to close the incision.
19. The method of paragraph 18, further including moving the tool assembly from the active position to the inactive position after the tool assembly has been inserted back into the body cavity.
20. The method of paragraph 19, wherein after rotating the closure device to position the suture needle beneath the second side of the incision, further including moving the tool assembly from the inactive position to the active position after the closure device is rotated to position the suture needle beneath the second side of the incision.

## Claims

1. An incision closure device comprising:
an elongate body defining a longitudinal axis and having a proximal portion and a distal portion, the elongate body defining a channel that extends from the proximal portion to the distal portion and supporting a shield;
a tool assembly supported on the distal portion of the elongate body, the tool assembly including a suture needle, a housing and first and second links, the suture needle having a proximal portion having a tip configured to penetrate tissue and a distal portion, the distal portion being pivotably secured to the first and second links of the tool assembly, the first and second links being pivotably secured to the housing;
an actuation member positioned within the channel of the elongate body and coupled to the first and second links such that the actuation member is movable from a retracted position to an advanced position to move the suture needle from an inactive position in which the tissue penetrating tip of the suture needle is positioned within the shield to an active position in which the tissue penetrating tip is positioned outwardly of the shield.

2. The incision closure device of claim 1, wherein the suture needle defines a longitudinal axis that is substantially parallel to the longitudinal axis of the elongate body in the inactive position and in the active position.

3. The incision closure device of claim 1 or claim 2, wherein each of the first and second links defines an elongate slot and the actuation member is coupled to the first and second links by pivot pins that are positioned within the elongate slots.

4. The incision closure device of claim 3, wherein the distal portion of the actuation member includes a bracket having spaced side walls defining a channel, the first and second links being received within the channel.

5. The incision closure device of claim 4, wherein the proximal portion of the suture needle includes a yoke defining a yoke channel, the first and second links being secured within the yoke channel.

6. The incision closure device of any preceding claim, further including a handle assembly, wherein the elongate body extends distally from the handle assembly.

7. The incision closure device of claim 6, wherein the handle assembly includes a drive member and a trigger, the drive member being coupled to a proximal end of the actuation member and the trigger being coupled to the drive member such that actuation of the trigger causes movement of the actuation member between the retracted and advanced positions.

8. The incision closure device of claim 7, further including a biasing member positioned to engage the drive member to urge the actuation member towards the retracted position.

9. The incision closure device of claim 8, wherein the handle assembly includes a housing and the housing defines a recess that receives the drive member.

10. The incision closure device of claim 9, wherein the housing includes a detent positioned within the recess and the drive member includes first and second concavities, the first concavity being positioned to receive the detent when the actuation member is in the retracted position and the second concavity being positioned to receive the detent when the actuation member is in the advanced position.

11. The incision closure device of claim 10, wherein the trigger includes first and second triggers that are coupled to the drive member and movable in a scissor-like motion to move the actuation member from the retracted position to the advanced position.

12. The incision closure device of claim 11, wherein the triggers are coupled to the drive member by first and second drive links.

13. A tool assembly comprising:
a suture needle, a housing, and first and second links, the suture needle having a proximal portion having a tip configured to penetrate tissue and a distal portion, the distal portion pivotably secured to the first and second links, the first and second links being pivotably secured to the housing, the first and second links movable from a first position to a second position to move the suture needle from an inactive position in which the tissue penetrating tip of the suture needle is positioned within the shield to an active position in which the tissue penetrating tip is positioned outwardly of the shield; preferably wherein the proximal portion of the suture needle includes a yoke defining a yoke channel, the first and second links secured within the yoke channel.

14. The tool assembly of claim 13, wherein the suture needle defines a first axis in the active position and a second axis in the inactive position, the first axis being parallel to the second axis; and/or wherein the first and second links each define an elongated slot, the elongated slots receiving a pivot member that is secured to the housing.

15. The tool assembly of claim 13 or claim 14, wherein the suture needle includes a suture opening.
